# EUROPEAN PATENT SPECIFICATION

(11) **EP 1 148 793 B1**
(45) Date of publication and mention of the grant of the patent: **13.08.2003**
(21) Application number: 00902518.0
(22) Date of filing: 03.02.2000
(51) Int. Cl.: A23L 1/30, A61K 9/10

(54) **METHOD OF PREPARING MICROPARTICLES OF PHYTOSTEROLS OR PHYTOSTANOLS**
VERFAHREN ZUR HERSTELLUNG VON PHYTOSTEROL- ODER PHYTOSTANOL-MIKROPARTIKELN
PROCEDE DE PREPARATION DE MICROPARTICULES CONSTITUEES DE PHYTOSTEROLS OU DE PHYTOSTANOLS

(30) Priority: 03.02.1999 US 243877
(43) Date of publication of application: 31.10.2001
(73) Proprietor: Forbes Medi-Tech Inc., Vancouver, British Columbia V6C 2T8 (CA)
(72) Inventor: ZAWISTOWSKI, Jerzy, Port Moody, British Columbia V3H 4K6 (CA)
(74) Representative: Jones, Helen Marjorie Meredith
(86) International application number: CA0000096
(87) International publication number: WO00045648

(56) References cited:
- EP-A- 0 897 671
- WO-A-98/13023
- WO-A-99/63841
- GB-A- 934 686
- US-A- 3 881 005
- US-A- 4 195 084
- DATABASE WPI Section Ch, Week 199219 Derwent Publications Ltd., London, GB; Class B01, AN 1992-154549 XP002139316 & JP 04 091026 A (GREEN CROSS CORP), 24 March 1992 (1992-03-24)
- PATENT ABSTRACTS OF JAPAN vol. 1998, no. 14, 31 December 1998 (1998-12-31) & JP 10 259114 A (SHISEIDO CO LTD), 29 September 1998 (1998-09-29)

## Description

### Field of the Invention

The present invention relates to the field of phytosterol and phytostanol solutions, emulsions, dispersions and compositions and to the use of the foregoing in foods, beverages, pharmaceuticals and nutraceuticals.

### Background of the Invention

Phytosterols have received a great deal of attention due to their ability to decrease serum cholesterol levels when fed to a number of mammalian species, including humans. While the precise mechanism of action remains largely unknown, the relationship between cholesterol and phytosterols is apparently due, in part, to the similarities between the respective chemical structures (the differences occurring on the side chains of the molecules). It is assumed that phytosterol replaces cholesterol from the micellar phase thereby reducing its absorption.

Given that phytosterol in various combinations have been proven to have wide clinical and dietary applications in lowering total and low density lipoprotein cholesterol, the key problem now facing researchers in this field is the adaptation of the phytosterols and their hydrogenated counterparts, phytostanols for incorporation into delivery systems and the possible modification of phytosterols/stanols to enhance their efficacy. Studies have investigated how the form (for example crystalline, suspension, granular) in which the phytosterols are dosed impacts on their ability to lower serum cholesterol levels. Phytosterols are highly lipophilic, do not dissolve to any extent in the micellar phase in the digestive tract and are therefore not efficient at blocking cholesterol absorption. Oils and fats to a limited degree are capable of dissolving free phytosterols. Since only solubilized phytosterols inhibit the absorption of cholesterol, this "delivery" problem must be adequately addressed.

Early research focussed on grinding or dry milling the phytosterols in order to try and enhance solubility (US Patent Nos:3,881,005 and 4,195,084 both to Eli Lilly). In addition, researchers have looked to the esterification of phytosterols in order to enhance solubility in delivery systems. German Patent 2035069/January 29, 1971 (analgous to US Patent No. 3,751,569) describes the addition of phytosterol fatty acid esters to cooking oil. The significant drawback to this process, among others, is the use of non-food grade catalysts and reagents.

Similarly, the incorporation of physterols into delivery vehicles, whether they be food or pharmaceutical-based, for administration to humans has been fraught with difficulties. US Patent No. 4,588,717 to David E Mitchell Medical Research Institute discloses a vcitamin supplement with fatty acid esters of phytosterols. US Patent No. 5,270,041 to Marigen S.A. teaches the use of phytosterols, their esters and glucosides for the treatment of tumours. The preparation of this composition involves the use of hazardous chemical reagents which effectively precludes the phytosterol component's ready use in all but a limited pharmaceutical area.

WO9813023A discloses the preparation of microcrystalline plant steroids by means of wet grinding and homogenisation in a viscous syrup suspension comestible products comprising the microcrystalline phytosterols are described.

US 4195084 and US3881005 disclose microparticulate phytosterols made by grinding or air milling.

GB934686 discloses the preparation of microparticulate phytosterol in suspension by grinding using a disc mill.

WO9563841 (& EP1082026), which was not published at the priority date hereof, discloses phytosterols and phytostanols treated to an enhanced solubility, for instance by using a homogenizer.

JP-A-10-259114 discloses oil-in-water type emulsions of fats, for instance phytosterols, formed by means of a high pressure emulsifier. The particles size of the emulsion is in the range of 0.3 to 0.5 µm.

WO9963841A & EP0897671 disclose phytosterols having a particle size of less than 15 µm used in foods.

In view of the high dietary and pharmaceutical usefulness of phytosterols, it would be advantageous to have a simple, safe and effective means to incorporate these sterols into delivery vehicles, including foods, beverages, nutraceuticals and pharmaceuticals. It is an object of the present invention to obviate or mitigate the above disadvantages.

### Summary of the Invention

The present invention provides, in the first part, a method of preparing microparticles of one or more phytosterols, phytostanols or mixtures of both which comprises dispersing or suspending the phytosterols and/or phytostanols in a semi-fluid, fluid or iscous vehicle and then exposing the vehicle so formed comprising the dispersed or suspended phytosterol, phytostanol or mixture to impact forces created by high shear using one of the following: a microfluidizer, an air atomization nozzle, a pneumatic nozzle, a high shear mixer or a colloid mill to produce microparticles.

The present invention also provides, in a second part, a method of incorporating one or more phytosterols, phystanols or mixture of both into a delivery vehicle which comprises simultaneously introducing into a microfluidizer the phytosterols and/or stanols and the chosen delivery vehicle and operting the microfluidizer under suitable pressure.

The present invention further provides, in a third part, a composition comprising microparticles of one or more phytosterols, phytostanols or mixtures of both in a semi-fluid, fluid or viscous vehicle.

The present invention further provides foods, beverages, pharmaceuticals and nutraceuticals which comprise microparticles of physterols and/or phytostanols produced by one of the novel methods.

The compositions described herein are useful to prevent or treat primary or secondary dyslipidemias and atherosclerosis including coronary heart disease, peripheral vascular disease and strokes in humans and animals.

What this method of reducing phytosterols and/or stanols into substantially uniform submicron particles achieves is two-fold. Firstly, the efficacy of the phytosterols/stanols in preventing and treating primary and secondary dislipidemias and cardiovascular disease and in lowering serum cholesterol is enhanced by virtue of the more discrete particle size. Secondly, the ultimate dispersion or incorporation of the phytosterols/stanols into a delivery vehicle of choice is more uniform than has heretofore been achieved. There are many delivery vehicles into which the phytosterols/stanols may be incorporated in accordance with the methods of the present invention described below. Without limiting the generality of the foregoing, these delivery vehicles may include any edible oil or aqueous food, beverage, nutraceutical or pharmaceutical matrix.

### Preferred Embodiments of the Invention

In essence, the method of preparing microparticles of phytosterols and/or phytostanols using impact forces in accordance with the present invention produces uniform submicron particles which are highly suitable for therapeutic and dietary uses as is or, alternatively, they may be incorporated into other food, beverage, nutraceutical or pharmaceutical-based delivery systems. It has been found that the phytosterols/stanols so prepared have greater solubility, not only in oil-based delivery systems but in other media and aqueous systems which opens the door for a vast array of options for their administration, particularly in the area of foods and beverages.

### Impact Forces/Preparing Microparticles

Prior researchers have attempted to reduce the particle size of phytosterols/phytostanols (hereinafter collectively referred to as "phytosterols" unless otherwise indicated) by conventional grinding and milling. These techniques are cumbersome, expensive and, because dry milling generates high energy, the produced particles could form aggregates. In addition, producing uniform particle size distribution below 10um is difficult if not impossible using these conventional techniques. In contrast, the "impact" forces described and claimed within the scope of the present invention allow for the production of smaller and more uniform particle size, with all of the attendant advantages. In addition, these "impact forces" result in faster processing times, higher reproducibility from batch to batch and allows for the production of more uniform dispersions and emulsions.

A preferred means to reduce the particle size of the phytosterols is by shear forces, wherein the semi-fluid, fluid or viscous vehicle comprising the dispersed or suspended phytosterols is forced through an air-atomization or pneumatic nozzle or a microfluidlzer. Particle size reduction may also be achieved by steep shearing gradients in high-speed stirrers or colloid mills.

Micropartlcles of phytosterols having a wide range of shapes and sizes may be prepared in accordance with the present invention. Depending on the type of Impact force used, slightly different end products may be achieved. For example, when using an air-atomization nozzle, the air pressure and configuration of the nozzle effects the final microparticle size. Nonetheless, as used herein, the term microparticle shall refer to a solid particle typically ranging from about 1 to 1000 microns . Micrypartictes below 20 microns are most preferred for incorporation into foods, beverages and nutraceuticals. Although spherical particles are preferred for some applications (and are normally produced by air-atomization), it has been found that irregularly shaped microparticles of phytosterols are equally suitable for the ultimate incorporation into delivery vehicles.

### 1) Microfluidization

Microfluidization, or particle collision technology, achieves what traditional homogenizers, grinding mills and other equipment have failed adequately to do: create uniform dispersions, emulsions and the like comprising microparticulate phytosterols.

By way of comparison, traditional grinding mills have extensive limitations including contamination of products, scalability and control difficulties, extensive processing times and high support requirements. The alternative, homogenizer valves, which push fluids through a variable geometry spring-loaded valve, are also fraught with limitations including the requirement for a high volume through-put and low pressures (leading to variable particle size in end product).

In contrast, using particle collision technology as the "impact" force, the resultant particle size is smaller and more consistent due to the higher pressure attained in the microfluidization chamber up to 2.76·10⁸ Nw/m² compared to 0.69·10⁸-0.83·10⁸ Nw/m² in a standard homogenizer (up to 40,000 psi compared to 10,000-12,000 in a standard homogenizer).

Particle size reduction and the formation of dispersions, emulsions and other delivery vehicles or matrices comprising phytosterols and using microfluidization has not heretofore been explored or achieved. Herein lies the core of the present invention. Equipment for this purpose is commercially available from Microfluidics Corporation, Newton, Mass. (USA). Microfluidization employs the forces of shear, impact and collision to achieve these ends as described in detail below.

Two important features define the microfluidization apparatus:
i) an interaction chamber having liquid jet paths of fixed geometry; and
ii) an intensifier pump allowing delivery of the liquid to the interaction chamber at constant pressure.

The intensifier pump may be any high-pressure pump but it is most preferred to use an air-driven or electric-driven hydraulic pump. The interaction chamber is generally a ceramic block with a system of channels running therethrough. Under high pressure, which can incrementally and accurately be increased or decreased over a wide range simply by adding or subtracting pneumatic or hydraulic pressure, phytosterols and/or phytostanols, previously dissloved, dispersed or otherwise suspended in a liquid vehicle enter the chamber and are split into two or more streams. The streams are turned at right angles and impacted upon each other resulting in shear (laminar flow), turbulence and cavitation (vapour bubble implosion). This technique exposes each volume of fluid to forces which are relatively consistent throughout the entire process thereby producing microparticles of phytosterols and/or phytostanol of substantially uniform size and shape. In a preferred form the microfluidizer is operated between 1.03·10⁸ and 1.58·10⁸ Nw/m² (15,000 and 23,000 psi.). Increasing the number of passes through the chamber further decreases the particle size.

The microparticulate phytosterol and /or phytostanols so formed may be used without further modification or adaptation and incorporated directly into foods, beverages, nutraceuticals and pharmaceuticals or, alternatively may be further treated (e.g. esterified and/or hydrogenated) and/or formed into other delivery vehicles such as emulsions, microemulsions, liposomes, hydrated lipid systems, cyclodextrin or bile acid complexes and the like prior to such incorporation.

Although it is known in the art to produce microparticles comprising various biological materials, what has not heretofore been achieved or appreciated is the formation of phytosterol and/or phytostanol microparticulates using microfluidization and the benefits afforded by this formation. US Patent No. 5,500,161 to Andrianov et al. describes a method of preparing hydrophobic polymeric microparticles suitable for encapsulating material such as proteins, liposomes and cells. One preferred means to coagulate the polymer with the material is via microfluidization. Similarly, US Patent No. 5,516,543 to Amankonan et al. discloses the preparation of oil-coated microparticulate gellan gum suitable for use as a fat replacer or extender.

Within the scope of the present invention, the vehicle into which the phytosterol and/or phytostanol component is dispersed or otherwise suspended may be any organic, inorganic or aqueous media or any food, beverage, nutraceutical or pharmaceutical matrix, including, but not limited to: all edible oils such as canola oil, soybean oil, corn oil, coconut oil, cottonseed oil, olive oil, palm oil, peanut oil, rapeseed oil, safflower oil, sesame oil and sunflower oil (vegetable oils and soybean oils being the most preferred) and the like, all fats, butter (including cocoa butter), lard, milk and other dairy beverages and all aqueous solutions, dispersions and suspensions including soy beverages, colas, juices and dietary supplement/meal replacement drinks.

### 2) Particle Size Reduction by other Shear Forces

Although microfluidization is the most preferred method, microparticles of phytosterols may also be prepared within the scope of the present invention by using air-atomization or pneumatic nozzles or by using high shear mixers or colloid mills. Colloid mills force liquid through very small clearances (for example 1/1000 of an inch) between two opposing phases known as the rotor and the stator, thereby producing microparticles by shear energy. The preferred equipment for such shearing force is an air atomizer sold by Turbotak Corporation (Ottawa) or an ultrasonic spray nozzle such as Sonimist sold by Medsonic Inc. (Farmingdale NY).

### Incorporation Phytosterols into Delivery Vehicles

In a further aspect of the present invention, there is provided a method by which one or more phytosterols, phytostanols or mixtures or both may be incorporated into a suitable delivery system using microfluidization technology. A composition of the phytosterols and/or phytostanols is simultaneously introduced into the interaction chamber of a microfluidizer along with a stream comprising the delivery vehicle into which it is desired that the phytsterol/phytostanol be incorporated. The microfluidizer is operated at the desired pressure and the resultant product is a vehicle into which microparticulates of the phytosterol/stanol have been uniformly and evenly distributed. As described above, when the particle size of the phytosterols/stanols is decreased, the efficacy and stability of delivery vehicle is enhanced. The delivery vehicle may be any organic, inorganic or aqueous media or any food, beverage, nutraceutical or pharmaceutical matrix, including, but not limited to any edible oil such as canola oil, soybean oil, corn oil, coconut oil, cottonseed oil, olive oil, palm oil, peanut oil, rapeseed oil, safflower oil, sesame oil and sunflower oil (vegetable oils and soybean oils being the most preferred); any fat-based food matrix such as milk, cream and other dairy products, lard, butter (including cocoa butter) or animal fat; or any beverage such as colas, soft drinks, juices, soy beverages, and dietary supplement/meal replacement drinks. This list is not intended in any way to be exhaustive.

Furthermore, in another embodiment of this invention, when phytosterols and/or phytostanols are incorporated into a delivery vehicle or "base matrix" using microfluidization technology, this base matrix can then further be use to prepare, in particular, other foods and beverages, or alternatively pharmaceuticals. For example, phytosterols and/or stanols may be incorporated at varying concentrations, but most preferably at concentrations up to 12%, into milk using microfluidizing technology thereby creating a stable dispersion. The milk so prepared is then a suitable base for making other products such as ice cream, cream for butter and cheeses and yoghurt and other dairy products. When the base matrix is fat like cocoa butter, the phytosterols an/or phytostanols may be incorporated therein using microfluidizing technology and subsequently used to make chocolate and other confections. When the base matrix is a fat or fat blend, for example comprising lard, lard flakes, palm oil, palm kernel oil, cottonseed oil, cocnut oil, soybean oil, corn oil, rapeseed oil or the like, an emuslion is formed using the method of the present invention which subsequently can be used to prepare cereal bars. Furthermore, when the base matrix is a fat or fat blend or edible oil, the product so formed by microfluidizing with phytosterols and/or phytostanols is equally suitable for many pharmaceutical applications, including the incorporation of the product into gel caps. The uses of microparticulate phytosterols/stanols are varied and accordingly, it is not intended that the present invention be limited to any particular base vehicle to the exclusion of others.

In addition, edible emulsions comprising phytosterols an/or phytostanols may be formed using the microfluidizing technology. For example, and as described further below, phytosterols and/or phytostanols can be emulsified into oils and fats and then subsequently used to produce dressings such as salad and vegetable dressings, mayonnaise, dairy and non-dairy spreads, chocolates and other confections and beverages.

In a preferred form, the incorporation of phytosterols and/or phytostanols into the base matrix or delivery vehicle is as follows: non-milled phytosterols and/or phytostanols in powder form, preferably of particle size around 100 um, are blended or suspended into the delivery vehicle (for example, fats, oils or aqueous solutions as described above) using a batch mixer, preferably a high shear mixer such as T50 Ultra Turrex. Subsequently, the blend is forced into a microfluidizer interaction chamber using a pump or compressed air. Microfluidization is performed under pressure of 1.03·10⁸ to 1.58·10⁸ Nw/m² (15,000 to 23,000 psi), most preferably around 1.38·10⁸ Nw/m² (20,000 psi). Several passes through the chamber may be required in order to achieve the preferred phytosterol/stanol particle size i.e. under 20 microns, most preferably in the range of 10-20 microns.

As used herein, the term phytosterol includes all phytosterols without limitation, for example: sitosterol campesterol, stigmasterol, brassicasterol, desmosterol, chalinosterol, poriferasterol, clionasterol and all natural or synthesized forms thereof, including isomers. The term "phytostanol" includes all hydrogenated (saturated) or substantially hydrogenated phytosterols and all natural or synthesized forms thereof, including isomers. It is to be understood that modifications to the phytosterols and/or phytostanols i.e. to include side chains, also falls within the purview of this invention. It is also to be understood that this invention is not limited to microparticulates of any particular one or combination of phytosterols and/or phytostanols. In other words, microparticulates of any phytosterol or phytostanol alone or in combination with other phytosterols and/or phytostanols in varying ratios as required may be formed in accordance with the present invention. For example, the composition referred to in US Patent Serial No. 5,770,749 to Kutney et al. (hereinafter called "Kutney et al." and incorporated herein by reference) may be formed into microparticulates or otherwise incorporated into a delivery system using a microfluidizer in accordance with the present invention.

Phytosterols and phytostanols may be procured from a variety of natural sources. For example, they may be obtained from the processing of plant oils (including aquatic plants) such as corn oil and other vegetable oils, wheat germ oil, soy extract, rice extract, rice bran, rapeseed oil, sesame oil, fish oils and other marine-animal oils. Without limiting the generality of the foregoing, phytosterols and/or phytostanols may be extracted from tall oil-derived pulping soap (a by-product of forestry practises) as described in Kutney et al.

In one embodiment of the present invention, the phytosterols and/or phytostanols are isolated from the source and formed into a solid powder through either precipitation, filtration and drying, spray drying, lyophilization or by other conventional work-up techniques. This powder form may then be incorporated into a base matrix or delivery vehicle using the microfluidizing technology as described above. In other words, this powder or alternatively, phytosterols/stanols directly from the source (e.g. vegetable oils) without any prior work-up techniques, may be either:
1) exposed to impact forces just to reduce the particle size of the constituent phytosterols/stanols; or
2) incorporated into a base matrix or delivery vehicle (e.g. milk, fat cream or the like) using microfluidizing technology, thereby not only reducing the particle size of the phytosterols/stanols but assisting in the uniform dispersion throughout the delivery vehicle; or
3) formed into emulsions using microfluidizing technology.

### Emulsions

Emulsions are finely divided or colloidal dispersions comprising two immiscible liquids or "phases", e.g. oil and water, one of which (the internal or discontinuous phase) is dispersed as droplets within the other (external or continuous phase). Thus, an oil-in-water emulsion consists of oil as the internal phase and water as the external or continuous phase, the water-in-oil emulsion being the opposite.

A wide variety of emulsified systems may be formed comprising phytosterols and/or stanols and using microfluidizing technology including standard emulsions and microemulsions.

Generally, emulsions comprise oil and water phases, emulsifiers, emulsion stabilizers, and optionally thickening agents, preservatives, colouring agents, flavouring agents, pH adjusters and buffers, chelating agents, vitamins, anti-foam agents, tonicity adjusters and anti-oxidants. Suitable emulsifiers include (wherein bracketed numerals refer to the preferred HLB value) include: anionic surfactants such as alcohol ether sulfates, alkly sulfates (30-40), soaps (12-20) and sulfosuccinates; cationic surfactants such as quarternary ammonium compounds; zwitterionic surfactants such as alkyl betaine derivatives; amphoteric surfactants sucha s faaty amine sulfates, difatty amine sulfates, difatty alkyl triethanolamine derivatives (16-17); and nonionic surfactants such as the polyglycol ether derivatives of aliphatic or cycloaliphatic alcohols, saturated fatty acids and alklyphenols, water-soluble polyethyleneoxy adducts onto polypropylene glycol and alkly polypropylene glycol, nonylphenol polyoxyethanols, castor oil polyglycol ethers, polyproplene/polyethylene oxide adducts, tributlyphenoxy-polyethoxyethoxyethanol, lanothin alcohols, polyethylated (POE) alkyl phenols (12-13), POE fatty esters poloxamers (7-19), POE glycol monoethers (13-16), polysorbates (17-19) and sorbitan esters (2-9). This list is not intended to be exhaustive as other emulsifiers are suitable.

Suitable emulsion stabilizers include, but are not limited to, lyophilic colloids such as polysaccarides, acacia, agar, alginic acid, carrageenan, guar gum, karaya gum, tragacanth, xanthan gum;, amphoterics (e.g. gelatin) and synthetic or semi-synthetic polymers (e.g. carbomer resins, celluloase thers and esters, carboxymethyl chitin, polyethylene glycol-n (ethylene oxide polymer H(OCH2CH2)nOH; finely divided solids including clays (e.g. attapulgite, bentonite, hectorite, kaolin, magnesium aluminum silicate and motmorillonite), microcrystalline cellulose oxides and hydroxides (e.g. aluminum hydroxide, magnesium hydroxide and silica); and cybotactic promoters/gellants (including amino acids, peptides, proteins, lecithin and other phospholipids and poloxamers.

Suitable anti-oxidants include: chelating agents, such as citirc acid, EDTA, phenlyalanine, phosphoric acid, tartaric acid and tryptophan; preferenatilly oxidized compounds such as ascorbic acid, sodium bisulfite and sodium sulfite; water soluble chain terminators such as thiols, and lipid soluble chain terminators such as alkyl gallates, ascorbyl palmitiate, t-butyl hydroquinone, butylated hydroxyanisole, butylated hydroxytoluene, hydroquinone, nordihydroguaiaretic acid and alpha-tocopherol. Suitable preservatives, pH adjusters and buffers, chelating agents, osmotic agents, colours and flavouring agents and their uses are well known in the art and can be added to the emulsions of the present invention as required.

It is important to note that the phytosterols and/or stanols may be either dispersed or suspended in *either* the oil or fluid/semi-fluid phase depending on the ultimate delivery vehicle sought to be created. For example, in preparing a beverage, the phytosterol/stanol may be suspended in the fluid portion and subsequently microfluidized. One mode to prepare a soy beverage is described in Example 5. Alternatively, in preparing a non-dairy spread, the phytosterol/stanol may be dispersed or suspended in a vegetable oil as described most preferably in Example 2 hereinbelow.

The preferred preparation of emulsions comprising phytosterols and/or stanols using microfluidizing technology in accordance with the present invention is as follows: the phytosterols and/or stanols are dispersed or suspended in an oil (or fluid) phase; the oil (or fluid) phase is then combined with a fluid or semi-fluid (or oil) phase along with an emulsifier, and any optional ingredients as listed above such as a thickening agent, to form a "blend"; the blend is then introduced into the microfluidizider at a pressure suitable to form and stabilize the emulsion. It is preferred that the oil phase comprises edible oils and fats, most preferably, vegetable oils. It is contemplated that many types of emulsions may be prepared using this process, including the formation of dairy and non-dairy spreads comprising one or more phytosterols and/or stanols.

In a further embodiment of the present invention, once such an emulsion is formed it may be encapsulated in a carbohydrate shell by a further pass through the microfluidizer as follows: blending the emulsion described above with a solution or suspension comprising one or more carbohydrates, preferably complex carbohydrates such as polysaccharides (for example: starch, inulin, glycogen) and/or one or more simple sugars such as glucose, fructose and the like―an appropriate suspension is corn syrup) and introducing this blend so formed into a microfluidizder at a suitable pressure. After subsequent spray drying, the resultant product is a core of phytosterol and fat or oil encapsulated in an outer carbohydrate shell.

### EXAMPLES

The following examples are intended merely to be illustrative and not limiting as to the scope of the present invention.

### Example 1: Preparation of yoghurt comprising microparticulates of phytosterols and/or phytostanols

A composition of plant sterols/stanols having campesterol (14.5%), campestanol (2.4%), beta-sitosterol (50.9%) and sitostanol (18.9%) (hereinafter referred to as "Phytrol™") was mixed with non-fat milk powder in the ratio of 1:7 to 1:8. About 6 L of milk mix was prepared from whole milk, skimmed milk and the Phytrol containing milk powder. Milk was standarized to 0.75-1% fat, 12-13% solids and 0.5-1% Phytrol using the Pearson's Square method (Hyde, KA and Rothwell, J. 1973, In Ice Cream, Churchill Livingstone Ltd., London U.K.). The milk mix was permitted to remain at room temperature for 30 minutes to re-hydrate the milk powder and then it was homogenized using a high-speed microfluidizer commercially available from Microfluidics Corporation, Newton, Mass. (USA). Next, milk was then pasteurized at 69°C (156°F) for 30 minutes (batch/vat), cooled to 44° C and held at this temperature for up to 30 minutes.

About 3% by weight of active yoghurt culture containing Lactobacillus bulgaricus and Streptococcus thermophilus in the ratio of !;! were carefully intrduced into the warm milk mix. After gentle mixing, the inoculated milk was distributed into 125G containers filling to near top. The containers were thermally sealed with aluminum leads and placed in an incubator (44°C) equipped with a good uniform air circulator and temperature controller. Filled containers were permitted to remain at 44°C for 3-5 hours, until a firm, smooth gel was formed. During incubation, pH was monitored periodically. When pH reached about 4.5, the yoghurt was withdrawn from the incubator, chilled quickly and stored at 4°C.

### Example 2: Preparation of a Vegetable Spread/Emulsion comprising microparticulates of phytosterols and/or phytostanols

A mixture of soybean oil and palm oil with Phytrol in the concentration range of 50-80% can be used to develop an emulsion. A small portion of hydrogenated vegetable oil (2-5%) can be added in order to obtain the desired texture. Two types of emulsions are possible: oil-in-water, which is preferable for the development of the low-fat spread and water-in-oil, which is preferable for some other applications. Appropriate emulsifiers or stabilizing agents such as lecithin, polysorbates and lactylates are used to stabilize the emulsion. Thickening agents such as gums (xanthan gum, locust bean gum, guar gum etc..), gelatin, pectins, and agar may also be added. To colour the spread, beta-carotene, caramel colour and FD&C yellow dye may be used. Furthermore, enriching the oil phase with vitamins A and D as well as with essential polyunsaturated fatty acids is possible.

A spread composition is as follows:

| | |
|---|---|
| Vegetable oil (liquid) | 50-80% |
| Vegetable, saturated fat | 0-5% |
| Phytrol | 9-15% |
| Emulsifier | 0.2-1% |
| Thickening agent | 0-10% |
| Butter Flavour, colourant, salt | various, as required |
| Water | to 100% |

All of the ingredients are blended in a stainless steel vessel equipped with a high sheer batch mixer such as the T50 Ultra Turrex. After blending is completed, and the mix has the consistency of heavy cream, the blend is tempered to a consistency of margarine by letting it sit for a couple of hours. To stabilize the emulsion and concomittantly to reduce the particle size of the phytrol component, the spread is homogenized in a microfluidizer.

### Example 3: Preparation of a Cereal Bar comprising microparticulates of phytosterols and/or phytostanols

It has been found by the applicants herein that Phytrol can be dispersed in fat up to 27% (and possibly more). For this reason, cereal bars having fat-based binders have been investigated. In this example, Phytrol is dispersed in fat to form a continuous emulsion. This fat component is then combined with carbohydrates and optionally with other ingredients to form a binder suitable to maintain the strength and elastic properties of the cereal bar.

### a) Binder

Generally, the fat-binder composition in cereal bars ranges from about 20-85% fat, and 20-60% carbohydrates by weight. The strength of the cereal bar is improved with the addition of up to 1% monoglycerides and diglycerides, however, since they have relatively high melting points compared to triglycerides, they should be used only in small proportions. Optionally, various emulsifiers, film formers (e.g. sodium caseinate or alternatively egg albumin, soy protein), colour and flavour components, vitamins and minerals may be added.

**A binder composition is as follows:**

| | |
|---|---|
| Phytrol containing fat | 40% |
| Sucrose | 22% |
| Water | 28% |
| Sodium Caseinate | 5% |
| Lecithin | 2% |
| Glycerin | 3% |

These ingredients are mixed at room temperature or added to boiled sucrose in water. Mixing is carried out vigorously using a suitable mixer (e.g. Hobart mixer) with the aim being to disperse the fat globules (discontinous pahse) in the film former/sucrose syrup (continuous phase). During this mixing process, fat is encapsulated. To determine whether this process is complete, place one drop of the dispersion in water at 60°C. If fat is released, mixing is not complete and should be continued.

### a) cereal bar

Any combination of oats, crisp cereals (corn and wheat flakes, Rice Knspies™), nuts, raisins and fruits, in various proportions comprise the "edible particles". All edible particles should be ready-to-eat. Cereals can be extruded, toasted or roasted in unsaturated oil such as soy or canola oil.

| **Composition of cereal bar** | |
|---|---|
| Binder (with Phytrol) | 40% |
| Edible particles | 55% |
| Water | 5% |

| Edible Particles: | |
|---|---|
| Rolled oats | 40% |
| Crisped rice | 15% |
| Puffed barley | 15% |
| Dried Apple Dices | 15% |
| Shredded Coconut | 7.5% |
| Raisins | 7.5% |

All of these ingredients are mixed thoroughly in a Hobart Mixer equipped with a kneeling device and a rotating bowl. The binder may be heated up to 40-50°C and placed first in the bowl followed by the other ingredients. Thorough mixing without causing size reduction of the edible particles should be used the criterion to set up time of mixing. After mixing is complete, mixed material is placed in a forming mold (10x50x0.6 cm) and pressed with a roller. After removal from the mold, it is cut into 4x10 cm ready-to-eat cereal bars. After forming and cutting, the bars may be single or double enrobed using dairy-based or chocolate cover. Bars should be stabilized at 10°C for 15-20 minutes before packing.

### Example 4: Preparation of Chocolate Confection comprising microparticulates of phytosterols and/or phytostanols

Chocolate is a dispersion of sugar and cocoa particles in a continuous phase of cocoa butter. The solid particles should generally be less than 20 um in diameter for the chocolate to have a smooth texture.

To make Phytrol-containing chocolate, Phytrol can either be mixed with cocoa particles (having undergone some impact procedure as described herein in order to reduce particle size) and used as such or alternatively Phytrol can be incorporated into cocoa butter using microfluidizing technology. Plain, white or milk chocolate can be made.

In the first approach, microparticulate Phytrol is mixed with cocoa powder, sugar, milk powder, emulsifier (soy lecithin), a film former and flavour agents (e.g. natural or artificial vanillin flavour). The dry mixing is conducted using a batch mixer such as T50 Ultra Turrex. Susequently, cocoa butter and milk are added and the formulation mixed thoroughly. After mixing, chocolate mass is tempered and used for molding.

In the second approach, Phytrol is incorporated directly into the cocoa butter. Cocoa butter is mixed with non-milled Phytrol powder and then passed through a microfluidizer (M-110Y Microfluidics International Co., Newton , Mass. USA) until the particle size is in the range of 10-20 microns using the procedure described in Example 2.

Once Phytrol-containing Butter is produced it is then used to prepare chocolate.

### Example 5: Preparation of Soy Drink comprising microparticulates of phytosterols and/or phytostanols

Soy drink is made of whole soybeans with filtered or purified water. It may contain added calcium, vitamin D, vitamin B-12 and natural or artificial flavour. In this example, soy drinks are enriched with Phytrol.

Phytrol is mixed with the soy drink of choice in the concentration ranges of 0.5-6% using a batch mixer (T50 Ultra Turrex). Samples are then submitted to the microfluidizer and emulsified.

## Claims

1. A method of preparing microparticles of one or more phytosterols, phytostanols or mixtures of both which comprises:
a) dispersing or suspending the phytosterols or phytostanols or mixtures of both in a semi-fluid, fluid or viscous vehicle; and
b) exposing the vehicle comprising the dispersed or suspended phytosterols, phytostanols or mixtures of both so formed to impact forces created by high shear using one of the following: a microfluidizer, an air atomization nozzle, a pneumatic nozzle, a high shear mixer or a colloid mill.

2. The method of claim 1 wherein the vehicle is an organic, inorganic or aqueous media.

3. The method of claim 1 wherein the vehicle is a food, beverage or nutraceutical matrix selected from the group consisting of edible oils, fats, milk, creams, and aqueous solutions and suspensions.

4. The method of claim 1 wherein the phytosterols are selected from the group consisting of sitosterol, campesterol, stigmasterol, brassicasterol, desmosterol, chalinosterol, poriferasterol, clionasterol and all natural or synthesized or isomeric or saturated forms and all derivatives thereof.

5. The method of claim 1 wherein the impact force is created using a microfluidizer.

6. The method of claim 1 wherein the impact of force is created by an air atomization nozzle.

7. The method of claim 1 wherein the impact force is created by a pneumatic nozzle.

8. A method of incorporating one or more phytosterols, phytostanols or mixtures of both into a delivery vehicle which comprises:
a) simultaneously introducing a solution of the phytosterols and/or phytostanols and the desired delivery vehicle into a microfluidizer; and
b) operating the microfluidizer at a suitable pressure.

9. The method of claim 8 wherein the delivery vehicle is selected from the group consisting of all edible oils, fats, milk, creams and all inorganic or organic aqueous solutions or suspensions.

10. The method of claim 8 wherein the delivery vehicle is a vegetable oil.

11. The method of claim 8 wherein the delivery vehicle is a fat.

12. The method of claim 8 wherein the delivery vehicle is cocoa butter.

13. A method of preparing an emulsion comprising one or more phytosterols, phytostanols or mixtures of both which comprises:
a) dispersing or suspending the phytosterols or phytostanols or mixtures of both in an oil phase;
b) combining the oil phase so formed and a suitable semi-fluid or fluid phase to form a blend;
c) introducing the blend into a microfluidizer; and
d) operating the microfluidizer under suitable pressure.

14. The method of claim 13 wherein the oil phase is selected from the group consisting of one or more edible oils and fats.

15. The method of claim 13 wherein the oil phase is vegetable oil.

16. The method of claim 13 wherein the semi-fluid phase comprises any organic, inorganic or aqueous media.

17. The method of claim 13 additionally comprising the steps of blending the emulsion so formed with a carbohydrate solution to form a second blend and introducing this second blend into a microfluidizer under suitable pressure.

18. A composition comprising microparticles of one or more phytosterols, phytostanols or mixtures of both wherein the microparticles are obtainable by the method of any of claims 1, 8 or 13.

19. The composition of claim 18 wherein substantially all of the microparticles are less than 20 microns.

20. The composition of claim 18 additionally comprising a delivery vehicle selected from the group consisting of all edible oils, fats, milk, creams and all inorganic or organic aqueous solutions, dispersion and suspensions.

21. A composition according to claim 18 which is an emulsion.

22. A composition according to claim 18 which is a dispersion.

23. A composition according to claim 18 which is a fat-based comestible.

24. A composition according to claim 18 which is a cereal-based comestible.

25. A composition according to claim 18 which is a dairy-based comestible.

26. A composition according to claim 18 which is a chocolate confection.

## Patentansprüche

1. Verfahren zur Herstellung von Mikroteilchen von einem oder mehreren Phytosterolen, Phytostanolen oder Mischungen von beiden, welches umfasst:
a) Dispergieren oder Suspendieren der Phytosterole oder Phytostanole oder der Mischungen von beiden in einem semifluiden, fluiden oder viskosen Vehikel und
b) Aussetzen des Vehikels umfassend die dispergierten oder suspendierten Phytosterole, Phytostanole oder Mischungen von beiden, das so gebildet ist, einer Stoßkraft, erzeugt durch eine hohe Scherbeanspruchung unter Verwendung einer der folgenden: ein Mikrofluidizer, eine Luftzerstäubungsdüse, eine pneumatische Düse, ein Mischer mit hoher Scherung oder eine Kolloidmühle.

2. Verfahren nach Anspruch 1, worin das Vehikel ein organisches, anorganisches oder wässriges Medium ist.

3. Verfahren nach Anspruch 1, worin das Vehikel ein Nahrungsmittel, ein Getränk oder eine nutraceutische Matrix, ausgewählt aus der Gruppe bestehend aus Speiseölen, Fetten, Milch, Cremes und wässrigen Lösungen und Suspensionen ist.

4. Verfahren nach Anspruch 1, worin die Phytosterole ausgewählt sind aus der Gruppe bestehend aus Sitosterol, Campesterol, Stigmasterol, Brassicasterol, Desmosterol, Chalinosterol, Poriferasterol, Clionasterol und allen natürlichen oder künstlichen oder isomeren oder gesättigten Formen und allen Derivaten davon.

5. Verfahren nach Anspruch 1, worin die Stoßkraft unter Verwendung eines Mikrofluidizers erzeugt wird.

6. Verfahren nach Anspruch 1, worin die Stoßkraft durch eine Luftzerstäubungsdüse erzeugt wird.

7. Verfahren nach Anspruch 1, worin die Stoßkraft durch eine pneumatische Düse erzeugt wird.

8. Verfahren zum Einverleiben von einem oder mehreren Phytosterolen, Phytostanolen oder Mischungen von beiden in ein Verabreichungsvehikel, welches umfasst:
a) gleichzeitiges Einleiten einer Lösung der Phytosterole und/oder der Phytostanole und des gewünschten Verabreichungsvehikels in einen Mikrofluidizer und
b) Betreiben des Mikrofluidizers bei einem geeigneten Druck.

9. Verfahren nach Anspruch 8, worin das Verabreichungsvehikel ausgewählt ist aus der Gruppe bestehend aus allen Speiseölen, Fetten, Milch, Cremes und allen anorganischen oder organischen wässrigen Lösungen oder Suspensionen.

10. Verfahren nach Anspruch 8, worin das Verabreichungsvehikel ein pflanzliches Öl ist.

11. Verfahren nach Anspruch 8, worin das Verabreichungsvehikel ein Fett ist.

12. Verfahren nach Anspruch 8, worin das Verabreichungsvehikel Kakaobutter ist.

13. Verfahren zur Herstellung einer Emulsion, umfassend ein oder mehrere Phytosterole, Phytostanole oder Mischungen von beiden, welches umfasst:
a) Dispergieren oder Suspendieren der Phytosterole oder Phytostanole oder der Mischungen von beiden in einer Ölphase,
b) Vereinen der so gebildeten Ölphase mit einer geeigneten semifluiden oder fluiden Phase, um eine Mischung zu bilden,
c) Einleiten der Mischung in einen Mikrofluidizer und
d) Betreiben des Mikrofluidizers unter einem geeigneten Druck.

14. Verfahren nach Anspruch 13, worin die Ölphase ausgewählt ist aus der Gruppe bestehend aus einem oder mehreren Speiseölen und Fetten.

15. Verfahren nach Anspruch 13, worin die Ölphase ein pflanzliches Öl ist.

16. Verfahren nach Anspruch 13, worin die semifluide Phase ein organisches, anorganisches oder wässriges Medium umfasst.

17. Verfahren nach Anspruch 13, das zusätzlich die Schritte des Mischens der so gebildeten Emulsion mit einer Kohlenhydratlösung, um eine zweite Mischung zu bilden, und des Einleitens dieser zweiten Mischung in einen Mikrofluidizer unter einem geeigneten Druck umfasst.

18. Zusammensetzung umfassend Mikroteilchen von einem oder mehreren Phytosterolen, Phytostanolen oder von Mischungen von beiden, worin die Mikroteilchen durch ein Verfahren nach irgendeinem der Ansprüche 1, 8 oder 13 erhältlich sind.

19. Zusammensetzung nach Anspruch 18, worin im wesentlichen alle Mikroteilchen kleiner als 20 Mikron sind.

20. Zusammensetzung nach Anspruch 18, die zusätzlich ein Verabreichungsvehikel umfasst, das aus der Gruppe bestehend aus allen Speiseölen, Fetten, Milch, Cremes und allen anorganischen oder organischen wässrigen Lösungen, Dispersionen und Suspensionen ausgewählt ist.

21. Zusammensetzung nach Anspruch 18, welche eine Emulsion ist.

22. Zusammensetzung nach Anspruch 18, welche eine Dispersion ist.

23. Zusammensetzung nach Anspruch 18, welche ein Nahrungsmittel auf Fettbasis ist.

24. Zusammensetzung nach Anspruch 18, welche ein Nahrungsmittel auf Getreidebasis ist.

25. Zusammensetzung nach Anspruch 18, welche ein Nahrungsmittel auf Milchproduktbasis ist.

26. Zusammensetzung nach Anspruch 18, welche ein Schokoladenkonfekt ist.

## Revendications

1. Procédé de préparation de microparticules constituées d'un ou plusieurs phytostérols, phytostanols ou de mélanges des deux, lequel comprend :
a) de disperser ou de mettre en suspension les phytostérols ou les phytostanols ou les mélanges des deux dans un véhicule semi-fluide, fluide ou visqueux ; et
b) d'exposer le véhicule comprenant les phytostérols, les phytostanols ou les mélanges des deux dispersés ou mis en suspension ainsi formé à des forces de collision créées par un cisaillement élevé en utilisant un des appareils suivants : un microfluidiseur, une buse de pulvérisation à l'air, un gicleur pneumatique, un mélangeur à fort cisaillement ou un broyeur de colloïdes.

2. Procédé de la revendication 1 dans lequel le véhicule est milieu organique, inorganique ou aqueux.

3. Procédé de la revendication 1 dans lequel le véhicule est une matrice à base d'un aliment, d'une boisson ou d'un alicament choisie dans le groupe constitué des huiles comestibles, des matières grasses, du lait, des crèmes et des solutions et suspensions aqueuses.

4. Procédé de la revendication 1 dans lequel les phytostérols sont choisis dans le groupe constitué du sitostérol, du campestérol, du stigmastérol, du brassicastérol, du desmostérol, du chalinostérol, du poriférastérol, du clionastérol et de toutes les formes saturées ou isomériques ou naturelles ou synthétisées et de tous les dérivés de ceux-ci.

5. Procédé de la revendication 1 dans lequel la force de collision est créée en utilisant un microfluidiseur.

6. Procédé de la revendication 1 dans lequel la force de collision est créée au moyen d'une buse de pulvérisation à l'air.

7. Procédé de la revendication 1 dans lequel la force de collision est créée au moyen d'un gicleur pneumatique.

8. Procédé d'incorporation d'un ou plusieurs phytostérols, phytostanols ou de mélanges des deux dans un véhicule de libération lequel comprend :
a) d'introduire simultanément une solution des phytostérols et/ou des phytostanols et le véhicule de libération souhaité dans un microfluidiseur ; et
b) de faire fonctionner le microfluidiseur à une pression appropriée.

9. Procédé de la revendication 8 dans lequel le véhicule de libération est choisi dans le groupe constitué des huiles comestibles, des matières grasses, du lait, des crèmes et de toutes les solutions ou suspensions aqueuses inorganiques ou organiques.

10. Procédé de la revendication 8 dans lequel le véhicule de libération est une huile végétale.

11. Procédé de la revendication 8 dans lequel le véhicule de libération est une matière grasse.

12. Procédé de la revendication 8 dans lequel le véhicule de libération est du beurre de cacao.

13. Procédé de préparation d'une émulsion comprenant un ou plusieurs phytostérols, phytostanols ou des mélanges des deux, lequel comprend :
a) de disperser ou de mettre en suspension les phytostérols ou les phytostanols ou les mélanges des deux dans une phase huileuse ;
b) de réunir la phase huileuse ainsi formée et une phase semi-fluide ou fluide appropriée pour former un mélange ;
c) d'introduire le mélange dans un microfluidiseur ; et
d) de faire fonctionner le microfluidiseur sous une pression appropriée.

14. Procédé de la revendication 13 dans lequel la phase huileuse est choisie dans le groupe constitué d'une ou plusieurs huiles et matières grasses comestibles.

15. Procédé de la revendication 13 dans lequel la phase huileuse est de l'huile végétale.

16. Procédé de la revendication 13 dans lequel la phase semi-fluide comprend n'importe quel milieu organique, inorganique ou aqueux.

17. Procédé de la revendication 13 comprenant en plus les étapes consistant à mélanger l'émulsion ainsi formée avec une solution de carbohydrate pour former un deuxième mélange et introduire ce deuxième mélange dans un microfluidiseur sous une pression appropriée.

18. Composition comprenant des microparticules d'un ou plusieurs phytostérols, phytostanols ou de mélanges des deux dans laquelle on peut obtenir les microparticules au moyen du procédé de l'une quelconque des revendications 1, 8 ou 13.

19. Composition de la revendication 18 dans laquelle pratiquement la totalité des microparticules sont inférieures à 20 microns.

20. Composition de la revendication 18 comprenant en plus un véhicule de libération choisi dans le groupe constitué de toutes les huiles comestibles, de toutes les matières grasses, du lait, de toutes les crèmes et de toutes les solutions, dispersions et suspensions aqueuses inorganiques ou organiques.

21. Composition selon la revendication 18 laquelle est une émulsion.

22. Composition selon la revendication 18 laquelle est un une dispersion.

23. Composition selon la revendication 18 laquelle est une denrée comestible à base de matière grasse.

24. Composition selon la revendication 18 laquelle est une denrée comestible à base de céréales.

25. Composition selon la revendication 18 laquelle est une denrée comestible à base de produits laitiers.

26. Composition selon la revendication 18 laquelle est une confiserie au chocolat.
